# NOUVEAU FASCICULE DE BREVET EUROPEEN

(11) **EP 0 131 319 B2**
(45) Date de publication et mention de la décision concernant l'opposition: **18.05.1994**
(45) Mention de la délivrance du brevet: 25.03.1987
(21) Numéro de dépôt: 84200801.3
(22) Date de dépôt: 06.06.1984
(51) Int. Cl.: C02F 3/28, C12P 5/02

(54) **Procédé et installation de digestion anaérobie**
Verfahren und Vorrichtung für anaerobe Gärung
Process and installation for anaerobic digestion

(30) Priorité: 01.07.1983 EP 83200980
(43) Date de publication de la demande: 16.01.1985
(73) Titulaire: Organic Waste Systems N.V.,in het kort: O.W.S. N.V., 2030 Antwerpen (BE)
(72) Inventeur: De Baere, Luc, B-9720 De Pinte (BE)
(74) Mandataire: Donné, Eddy

(56) Documents cités:
- DE-A- 3 015 239
- DE-C- 301 076
- DE-C- 601 668
- FR-A- 1 146 820
- FR-A- 2 309 492
- FR-A- 2 327 974
- FR-A- 2 480 778
- GB-A- 745 295
- GB-A- 1 110 352
- WASTEWATER TREATMENT PLANT DESIGN, 1977, pages 519-521, WPCF, Washington, D.C., US; "Anaerobic sludge digestion"

## Description

Il est déjà connu de produire du biogaz à partir de matières organiques et particulièrement d'immondices provenant de la collecte d'ordures ménagères au moyen d'une digestion anaérobie aidée éventuellement par une inoculation appropriée. Selon un tel procédé connu, les immondices provenant de la collecte sont d'abord broyées et triées pour en enlever les parties lourdes tels que métaux, verre, etc. Les parties légères, constituées essentiellement de matières organiques, sont mélangées avec de l'eau, des boues d'égout, du purin, etc. et/ou encore un autre inoculant approprié et soumises à un des procédés de digestion anaérobie pour produire du biogaz à partir d'une masse liquide, comprenant en général moins de 15 % de matière sèche.

Suivant un procédé décrit dans le brevet français FR-A1-2 327 974 un procédé de traitement d'ordures ménagères est connu dans lequel les ordures préalablement broyées sont arrosées à l'aide d'un inoculant liquide pendant la fermentation, l'inoculant liquide est recyclé. Ce procédé connu produit du méthane et nécessite une durée de fermentation de 15 à 30 journées. Au bout d'un temps de 15 journées, les ordures sont utilisables comme engrais, mais après 30 journées une telle utilisation n'est pas possible.

La présente invention a pour but un procédé de compostage anaérobie ou de digestion anaérobie à grande vitesse de matière organique d'apparence solide dont la dégradation presque totale par digestion anaérobie s'accomplit dans l'espace de quelques journées à trois mois. Un autre but de l'invention consiste en un procédé de digestion anaérobie de matières organiques non liquides dans lequel l'effet néfaste de l'acidification initiale de la matière organique est éliminé.

De plus, l'invention vise à obtenir un produit résiduel se présentant sous forme de compost stabilisé ou presque stabilisé, inodore ou presque inodore ne nécessitant pas de traitement ultérieur.

Suivant l'invention, un procédé de traitement de matières organiques comprenant une phase de préparation dans laquelle les matières organiques à soumettre à fermentation sont mélangées avec un inoculant approprié et introduites dans un digesteur anaérobie, est caractérisé en ce que les matières organiques sont mélangées à l'inoculant pour former une masse d'apparence solide qui est introduite dans le digesteur et en est extraite après une durée inférieure à 50 jours, en ce qu'une partie égale à au moins un tiers et de préférence égale à au moins deux tiers de la masse extraite du digesteur est recyclée vers son entrée et mélangée par malaxage à de la matière organique fraîche, au besoin prétraitée, soit par essorage, soit par humidification de manière que la masse introduite dans le digesteur soit une masse comprenant entre 25 et 45 % de matière sèche. La partie non recyclée de la masse sortant du fermenteur peut être utilisée comme compost humide, soit directement, éventuellement essoré ou séché, soit après passage dans un digesteur complémentaire.

L'invention est applicable, par exemple, à des matières organiques provenant de la collecte d'ordures ménagères, à des déchets de betteraves, déchets de pommes de terre, les parties solides venant d'un fluide de rejet après centrifugation ou pressage, etc. Dans le cas d'ordures ménagères, ces dernières sont broyées et délestées des parties lourdes telles que métaux, verre, etc. pour en isoler les parties composées presqu'exclusivement de matières organiques solides.

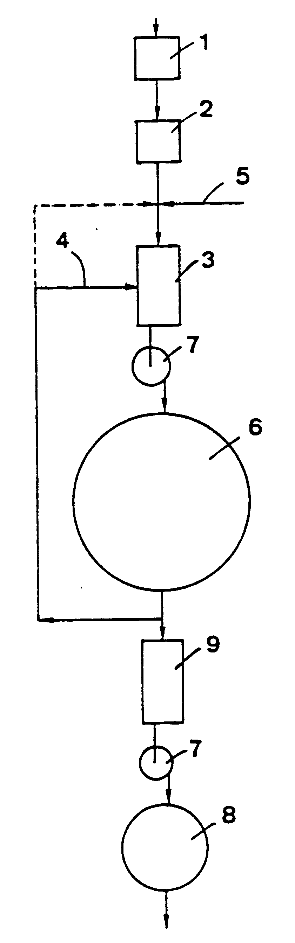

L'invention est décrite ci-dessous par rapport à un exemple de forme d'exécution représenté schématiquement dans l'unique figure du dessin annexé.

Dans le procédé schématisé au dessin annexé, des immondices provenant de la collecte d'ordures ménagères sont introduits dans un broyeur 1. A la sortie du broyeur 1, les parties lourdes, si elles existent, notamment les métaux, le verre, etc. peuvent éventuellement être séparées des parties organiques dans un séparateur 2. Il va de soi que dans le cas où les immondices sont purement organiques, par exemple des déchets de betteraves, un tel séparateur n'est pas indispensable.

Une séparation de certains composants des immondices peut avoir lieu aussi avant la phase de broyage, par exemple l'extraction de métaux ferro-magnétiques.

La masse en matière organique est introduite dans un mélangeur 3 simultanément avec une quantité déterminée d'une boue acétoclaste méthanigène ou boue d'inoculation amenée par un conduit 4 et éventuellement un apport d'eau, de préférence chaude, provenant d'un conduit 5. Le conduit 4 peut se raccorder soit en amont du mélangeur 3, comme représenté en pointillé, soit par une entrée spéciale du mélangeur 3, comme représenté en trait plein, La boue d'inoculation est ajoutée en très grande quantité, soit en une quantité au moins égale à la moitié et de préférence au double de la quantité de matière organique fraîche. L'utilisation de telles quantités de boues d'inoculation a pour effet d'éliminer les conséquences néfastes d'une acidification initiale de la matière organique fraîche grâce à l'inoculation intime et importante de la matière fraîche par la masse d'inoculation. Toutefois, l'utilisation permanente de telles quantités n'est possible que pour autant que la majeure partie de la boue d'inoculation soit constituée par de la boue de recyclage récoltée à la sortie de l'installation de digestion ou de compostage alimentée par le mélangeur 3. Cette installation de compostage est constituée par un digesteur 6 à productivité spécifique de gaz élevé. Pour un apport journalier de 100 t d'ordures délestées des parties lourdes, comprenant de 40 à 65 % de matière sèche, un apport d'eau, par exemple de 50 t, est nécessaire pour rendre la masse ainsi obtenue suffisamment humide pour être soumise à une digestion anaérobie à fermentation rapide. L'adjonction d'eau a lieu par malaxage dans le mélangeur 3. Le mélangeur 3 traite ainsi une masse dont le taux d'humidité est entre 55 et 75 %. Le mélangeur 3 reçoit par jour 150 t d'ordures humides d'apparence solide et par exemple 300 t de masse de recyclage. Une pompe 7, par exemple une pompe telle que l'on utilise pour pomper du ciment, envoie la masse de 450 t par jour sortant du mélangeur 3 dans le digesteur 6 d'une capacité d'au moins 10 fois l'arrivage journalier, par exemple 2000 m³. Dans ce digesteur se trouve, en régime, continuellement une masse d'environ 1 500 t dégageant une quantité importante de gaz méthane. La masse fournie par la pompe 7 est distribuée par exemple à la partie supérieure du digesteur 6, par exemple en forme de cylindre vertical et est extraite du fond de ce digesteur après un temps de passage de 3,5 jours environ en une proportion d'environ 405 t par jour, 45 t ayant été transformées en biogaz, principalement du méthane et récoltées sous cette forme pour un usage ultérieur. De ces 405 t, 300 t sont recyclées à l'entrée du mélangeur 3 *via* le conduit 4 tandis que 105 t seulement sont véhiculées vers la sortie. De cette manière, la durée moyenne de la digestion de la matière organique fraîche dans le digesteur 6 est d'environ 14 jours. Les 105 t de masse véhiculées vers la sortie peuvent être utilisées telles quelles comme engrais sous forme de compost, éventuellement après essorage et séchage réduisant son taux d'humidité en dessous de 50 %.

Toutefois, la masse d'apparence solide extraite du digesteur 6 subit de préférence une digestion finale dans un digesteur complémentaire 8 d'une capacité d'au moins 5 fois l'arrivage journalier par exemple 1 000 m³ d'où environ 100 t de la masse est extraite après un temps de passage d'environ 7 jours, 5 t ayant été transformées en biogaz. On arrive ainsi à transformer 100 t d'ordures ménagères sèches en 21 jours. La production de méthane pur au moyen du procédé décrit est très élevé et se situe aux environs de 250 m³ de CH₄ par tonne de matière sèche dégradable. La capacité des digesteurs 6 et 8 peut toujours rester inférieure à respectivement 50 et 25 fois l'arrivage journalier.

Pendant le transfert du digesteur 6 au digesteur 8, la masse peut traverser un mélangeur 9 dans lequel une aération convenablement dosée stimule une certaine production d'acides favorable à une digestion anaérobie ultérieure dans le digesteur 8.

Le produit sortant du digesteur 8 est complètement inactif et sans odeur; il forme un excellent compost.

Une partie du gaz récolté dans les digesteurs 6 et 8, après passage dans un compresseur, non représenté, peut être réinjecté dans le fond de chaque digesteur afin d'éviter un tassement trop dense de la masse d'apparence solide et de favoriser l'échappement du gaz produit. Le gaz recyclé peut servir de régulateur de la température dans le digesteur grâce à un échauffement ou à un refroidissement avant l'injection dans le fond du digesteur.

Il est possible aussi d'injecter une petite quantité d'air convenablement dosée en même temps qu'on injecte le gaz de recyclage afin de tenir compte d'une micro-aérophilie de la masse dans le fond du digesteur. Il est à observer ici qu'il ne s'agit pas d'un procédé de digestion aérobie, mais bien d'un procédé de digestion anaérobie dans lequel la quantité d'air admise doit être maintenue très faible. Dans ce cas, cette présence d'air stimule la dégradation de la masse dans les digesteurs et fait monter leur température.

La boue d'inoculation peut être constituée partiellement ou de préférence totalement de matière de recyclage. Toutefois, à condition qu'on puisse disposer en permanence d'une grande quantité de boue d'inoculation provenant d'une installation différente, par exemple d'une installation de traitement anaérobie de boues d'égout, la boue d'inoculation peut être constituée soit partiellement, soit totalement par celle fournie par cette installation différente. De toute manière, un apport de boue d'inoculation méthanigène provenant d'une source étrangère est utile et même indiqué lors du démarrage d'une installation décrite ci-dessus, jusqu'à ce que le pH dans les digesteurs 6 et 8 ait atteint des valeurs comprises entre 7 et 8.

En lieu et place de l'eau servant à l'humidification est amenée par le conduit 5, il est possible aussi d'utiliser un inoculant liquide provenant d'une source méthanigène.

Le procédé décrit est utilisable aussi bien dans le cas de réactions méthanigènes aux environs de 35 °C qu'aux environs de 60 °C. Le transport de la masse humide vers ou provenant des digesteurs 6 ou 8 peut être effectué de préférence par des pompes à béton.

L'eau récupérée lors de l'essorage du compost sortant du digesteur 6 ou du digesteur 8 peut être partiellement ou totalement recyclée *via* le conduit 5. Une limite peut s'imposer à ce recyclage si l'eau véhicule du sel qui pourrait agir en inhibiteur de la fermentation.

Le gaz produit dans l'installation peut être utilisé dans un groupe électrogène dont les gaz d'échappement peuvent être appliqués au séchage du compost sortant des digesteurs 6 ou 8.

## Revendications

1. Procédé de traitement de matières organiques comprenant une phase de préparation dans laquelle des matières organiques à digérer sont mélangées avec un inoculant approprié et introduites dans un digesteur anaérobie,
caractérisé en ce que les matières organiques sont mélangées à l'inoculant dans un mélangeur (3) pour former une masse d'apparence solide qui est introduite dans le digesteur (6) et en est extraite après une durée inférieure à 50 jours, en ce qu'une partie égale à au moins un tiers de la matière extraite du digesteur (6) est recyclée via un conduit (4) vers son entrée et mélangée par malaxage à une quantité de matière organique fraîche inférieure au double de cette quantité recyclée, la quantité de matière organique fraîche étant au besoin prétraitée soit par essorage, soit par humidification de manière que la masse à la sortie du mélangeur (3) présente un taux d'humidité entre 55 et 75%.

2. Procédé suivant la revendication 1, caractérisé en ce que la masse introduite dans le digesteur en est extraite après une durée inférieure à 25 jours et en ce qu'une partie égale à au moins deux tiers de matière extraite du digesteur (6) est recyclée vers son entrée et mélangée à une quantité de matière organique fraîche inférieure à la moitié de cette quantité recyclée.

3. Procédé suivant une des revendications précédentes, caractérisé en ce qu'il est suivi d'une digestion finale de la matière non recyclée.

4. Procédé suivant une des revendications précédentes, caractérisé en ce qu'une aération convenablement dosée des masses soumises à digestion anaérobie est prévue.

5. Procédé suivant une des revendications précédentes, caractérisé en ce que la matière extraite après digestion est soumise à un essorage et en ce que l'eau récupérée lors de l'essorage est recyclée, au moins partiellement, vers l'entrée du mélangeur (3) *via* un conduit (5).

6. Installation de traitement de matières organiques comprenant un digesteur anaérobie et des moyens pour mélanger les matières organiques à digérer avec un inoculant approprié, caractérisé en ce qu'un mélangeur (3) pour une masse de matière organique fraîche comprend des moyens pour maintenir le taux d'humidité de cette masse entre 55 % et 75 %, et au moins une entrée pour les matières organiques fraîches et une boue d'inoculation de recyclage, en ce que la sortie du mélangeur (3) est reliée *via* une pompe (7) à l'entrée d'un digesteur anaérobie (6) d'une capacité pour recevoir une masse entre 10 à 50 fois l'arrivage journalier moyen estimé de matière organique fraîche et en ce qu'un dispositif d'extraction extrait la masse digérée du fond du digesteur (6) et en envoie une partie *via* un conduit (4) suffisamment large pour véhiculer sensiblement plus qu'un tiers de la masse extraite vers l'entrée du mélangeur (3) et le reste vers la sortie du digesteur (6).

7. Installation suivant la revendication 5, caractérisée en ce que la sortie du digesteur (6) est reliée *via* une pompe (7) à l'entrée d'un deuxième digesteur (8) d'une capacité pour recevoir une masse entre 5 et 25 fois l'arrivage journalier moyen estimé de matière organique fraîche.

8. Installation suivant la revendication 7, caractérisée en ce qu'entre la sortie du digesteur (6) et l'entrée du deuxième digesteur (8) est disposé un mélangeur (9) dans lequel une aération convenablement dosée de la masse soumise à digestion anaérobie est prévue.

## Claims

1. Process for the treatment of organic materials comprising a preparation phase wherein organic materials to be digested are mixed with an appropriate inoculant and introduced into an anaerobic digester, characterised in that the organic materials are mixed with the inoculant in a mixer (3) to form a mass of solid appearance which is introduced into the digester (6) and is taken therefrom after a dwell time of less than 50 days, in that a portion equal to at least a third of the material taken from the digester (6) is recycled via a conduit (4) toits inlet and mixed by kneading with a quantity of fresh organic material less than twice this recycled quantity, the quantity of fresh organic material being, if necessary, pretreated either by drying or by moistening in such a manner that the mass at the outlet of the mixer (3) has a moisture content of between 55 and 75 %.

2. Process according to claim 1, characterised in that the mass introduced into the digester is taken therefrom after a period of less than 25 days, and in that a portion equal to at least two thirds of the material taken from the digester (6) is recycled to its inlet and mixed with a quantity of fresh organic material less than half of this recycled quantity.

3. Process according to one of the preceding claims, characterised in that it is followed by a final digestion of the non-recycled material.

4. Process according to one of the preceding claims, characterised in that a suitably dosaged aeration of the masses subjected to anaerobic digestion is provided.

5. Process according to one of the preceding claims, characterised in that the material taken out after digestion is subjected to a drying operation, and in that the water recovered in the drying operation is, at least partly, recycled to the inlet of the mixer (3) via a conduit (5).

6. Installation for the treatment of organic materials comprising an anaerobic digester and means for mixing the organic materials to be digested with an appropriate inoculant, characterised in that a mixer (3) for a mass of fresh organic material comprises means for keeping the moisture content of this mass between 55 % and 75 %, and at least one inlet for the fresh organic materials and a recycling inoculation sludge, in that the outlet of the mixer (3) is connected via a pump (7) to the inlet of an anaerobic digester (6) having a capacity for receiving a mass of between 10 and 50 times the estimated average daily intake of fresh organic material, and in that a discharging device takes the digested mass from the bottom of the digester (6) and sends a portion of this mass via a conduit (4) sufficiently wide to carry substantially more than a third of the discharged mass to the inlet of the mixer (3) and the remainder to the outlet of the digester (6).

7. Installation according to claim 6, characterised in that the outlet of the digester (6) is connected via a pump (7) to the inlet of a second digester (8) with a capacity able to receive a mass of between 5 and 25 times the estimated average daily intake of fresh organic material.

8. Installation according to claim 7, characterised in that between the outlet of the digester (6) and the inlet of the second digester (8) there is arranged a mixer (9) wherein a suitably dosaged aeration of the mass subjected to anaerobic digestion is provided.

## Patentansprüche

1. Verfahren zur Verarbeitung von organischen Stoffen, das eine Vorbereitungsphase aufweist, bei der zu vergärende organische Stoffe mit einem geeigneten Impfstoff gemischt werden und in einen anaeroben Gärbehälter gegeben werden,
dadurch gekennzeichnet, daß die organischen Stoffe in einem Mischer (3) mit dem Impfstoff gemischt werden, wobei eine feste Masse gebildet wird, die in den Gärbehälter (6) gegeben wird und nach einer Dauer von weniger als 50 Tagen wieder daraus entnommen wird, und daß ein Teil der aus dem Gärbehälter (6) entnommenen Stoffe, der mindestens einem Drittel dieser Stoffe entspricht, nach dem Eingang des Gärbehälters (6) über eine Rohrleitung (4) zurückgeführt wird, und durch Kneten mit einer Menge frischer organischer Stoffe gemischt wird, die kleiner als das Doppelte dieser zurückgeführten Menge ist, wobei die Menge frischer organischer Stoffe erforderlichenfalls entweder durch Trockenschleudern, oder durch Anfeuchten so vorbehandelt wird, daß die Masse am Ausgang des Mischers (3) einen Feuchtigkeitsgehalt zwischen 55 und 75% aufweist.

2. Verfahren gemäß Anspruch 1, dadurch gekennzeichnet, daß die in den Gärbehälter gegebene Masse nach einer Dauer von weniger als 25 Tagen wieder entnommen wird, und daß ein Teil der aus dem Gärbehälter (6) entnommenen Stoffe, der mindestens zwei Dritteln dieser Stoffe entspricht, nach dem Eingang dieses Gärbehälters (6) zurückgeführt wird, und mit einer Menge frischen organischen Stoffen gemischt wird, die kleiner als die Hälfte dieser zurückgeführten Menge ist.

3. Verfahren gemäß einem der vorhergehenden Ansprüche, dadurch gekennzeichnet, daß auf dieses Verfahren eine Endgärung der nicht zurückgeführten Stoffe folgt.

4. Verfahren gemäß einem der vorhergehenden Ansprüche, dadurch gekennzeichnet, daß eine geeignet dosierte Belüftung der der anaeroben Gärung unterworfenen Massen vorgesehen ist.

5. Verfahren gemäß einem der vorhergehenden Ansprüche, dadurch gekennzeichnet, daß die nach der Gärung entnommenen Stoffe einer Trockenschleuderung unterworfen werden, und daß das bei der Trockenschleuderung zurückgewonnene Wasser über eine Rohrleitung (5) zumindest teilweise nach dem Eingang des Mischers (3) zurückgeführt wird.

6. Vorrichtung zur Verarbeitung von organischen Stoffen, aus einem anaeroben Gärbehälter, und aus Mitteln zum Mischen der zu vergärenden organischen Stoffe mit einem geeigneten Impfstoff,
dadurch gekennzeichnet, daß ein Mischer (3) für eine Masse frischer organischer Stoffe Mittel aufweist, um den Feuchtigkeitsgehalt dieser Masse zwischen 55 und 75% zu halten, und außerdem mit mindestens einem Eingang für die frischen organischen Stoffe und für Recycling-Impfschlamm vorgesehen ist; daß der Ausgang des Mischers (3) über eine Pumpe (7) mit dem Eingang eines anaeroben Gärbehälters (6) verbunden ist, dessen Fassungsvermögen ausreicht, um eine Masse aufzunehmen, die der 10- bis 50-fachen geschätzten mittleren täglichen Anlieferung von frischen organischen Stoffen entspricht; und daß eine Entnahmevorrichtung die vergorene Masse von dem Boden des Gärbehälters (6) entnimmt, und einen Teil der entnommenen Masse über eine Rohrleitung (4), die für die Beförderung von wesentlich mehr als einem Drittel dieser Masse ausgelegt ist, nach dem Eingang des Mischers (3), und den Rest nach dem Ausgang des Gärbehälters (6) befördert.

7. Vorrichtung gemäß Anspruch 6, dadurch gekennzeichnet, daß der Ausgang des Gärbehälters (6) über eine Pumpe (7) mit dem Eingang eines zweiten Gärbehälters (8) verbunden ist, dessen Fassungsvermögen ausreicht, um eine Masse aufzunehmen, die der 5- bis 25-fachen geschätzten mittleren täglichen Anlieferung von frischen organischen Stoffen entspricht.

8. Vorrichtung gemäß Anspruch 7, dadurch gekennzeichnet, daß zwischen dem Ausgang des Gärbehälters (6) und dem Eingang des zweiten Gärbehälters (8) ein Mischer (9) angeordnet ist, in dem eine geeignet dosierte Belüftung der der anaeroben Gärung unterworfenen Masse vorgesehen ist.
